# EUROPEAN PATENT SPECIFICATION

(11) **EP 3 983 027 B1**
(45) Date of publication and mention of the grant of the patent: **14.10.2026**
(21) Application number: 20823470.8
(22) Date of filing: 13.06.2020
(51) Int. Cl.: A61M 25/00, A61M 25/10, A61B 8/12, A61B 8/08

(54) **INTRACARDIAC DELIVERY CATHETER**
INTRAKARDIALER EINFÜHRKATHETER
CATHÉTER DE POSE INTRACARDIAQUE

(30) Priority: 14.06.2019 US 201962861906 P; 09.04.2020 US 202063007792 P
(43) Date of publication of application: 20.04.2022
(73) Proprietor: TargetCath, Inc., Minneapolis, MN 55405 (US)
(72) Inventor: HAARSTAD, Philip J., Chanhassan, Minnesota 55317 (US); PANETTA, Carmelo J., Minneapolis, Minnesota 55405 (US)
(74) Representative: Thurgood, Alexander John
(86) International application number: PCT/US2020/037642
(87) International publication number: WO 2020/252416

(56) References cited:
- US-A- 5 102 416
- US-A- 5 308 317
- US-A- 5 571 161
- US-A- 5 571 161
- US-A- 5 879 499
- US-A1- 2001 023 332
- US-A1- 2005 159 645
- US-A1- 2008 097 402
- US-A1- 2011 021 911
- US-A1- 2011 098 561
- US-B1- 6 491 671

## Description

The present application claims priority to U.S. Serial No. 62/861,906 filed June 14, 2019 and entitled INTRACARDIAC IMAGING ASSEMBLY AND METHOD OF USE and U.S. Serial 63/007,792 filed April 9, 2020 entitled INTRACARDIAC DELIVERY CATHETER AND METHOD OF USE.

### BACKGROUND

Various cardiac treatment procedures require use of an imaging device during the treatment procedure. Intracardiac placement of the imaging or other therapeutic device into the pulmonary artery or other intracardiac treatment site can be difficult and during use, the device can prolapse making the treatment procedure more difficult and cumbersome. A number of devices are described in the prior art, such as US5571161A, US5879499A, US2011098561A1 and US2001023332A1.

US5571161A relates to a steerable guide catheter for the placement of electrical lead wires useful for cardiac pacemakers and other electrophysiology devices, including an atraumatic balloon at its distal end. The catheter has a proximal hemostatic seal and is axially splittable so that it may be removed over an electrical lead wire having an enlarged connector at its proximal end. The balloon is asymmetrically mounted to permit such splitting.

US5879499A relates to a process for forming a multi-lumen catheter which includes the steps of providing a multi-lumen first tube, winding a flexible filament onto the first multi-lumen tube, and positioning a second tube over the wound filament layer of the first tube. The second tube is melted and bonded to the first tube and an occluding member is mounted onto the second tube to occlude a patient's ascending aorta when the occluding member is expanded.

US2011098561A1 relates to a medical device including, e.g., an implantable medical lead or drug pump catheter, a guide member, or a sheath, which is delivered to a target location within a patient via a catheter capable of subselecting vessels or other cavities, and passages within the patient's body. The catheter employs an inflatable member that, when actuated, acts to deflect a medical device to direct that device into a vessel or other cavity or passage, in which the catheter is arranged. In some examples, the inflatable member acts to deflect the delivery catheter, which in turn necessarily deflects the implantable medical device arranged therein. In other examples, the inflatable member acts to deflect the medical device to turn the device into a vessel or other cavity, passage, or the like branching off of the vessel in which the catheter is arranged.

US2001023332A1 relates to an expandable lumen cannula which includes an elongate tube having a proximal end, a distal end, an intermediate flexible region, and a lumen therebetween. The cannula further includes a balloon occluder mounted on the distal end of the tube. The intermediate flexible region of the tube further includes an elongate generally cylindrical balloon disposed circumferentially about the flexible region which, upon inflation, expands the luminal diameter of the intermediate region.

### SUMMARY

The invention is defined by the claims.

The present application relates to an intracardiac delivery catheter for intracardiac placement of an imaging or treatment device. In the illustrated embodiments, the delivery catheter includes a flow assist balloon for intracardiac placement of the delivery catheter and a delivery lumen therethrough. In illustrated embodiments, the delivery lumen has a diameter dimension of at least 8F, 9F or 10F for insertion of an intracardiac echocardiography catheter or intracardiac echocardiography (ICE) catheter and guidewire. For example, in embodiments disclosed, the delivery lumen has a diameter dimension between 8F-14F or 9F-14F. The delivery lumen extends from an inlet at the proximal end of a catheter shaft to a distal end of the catheter shaft. In illustrated embodiments an outlet of the delivery lumen is distal from a flow assist balloon of the delivery catheter and is formed through an opened distal end of the catheter shaft and delivery lumen. Embodiments of the delivery catheter include a guide wire lumen in addition to the delivery lumen and an inflation lumen to expand the flow assist balloon for intracardiac placement of the delivery catheter. In an illustrated embodiment, the guide wire lumen is a shortened guide wire lumen.

Embodiments of the intracardiac delivery catheter include an asymmetric shaped flow assist balloon having an asymmetric shape in an inflated or expanded profile. As disclosed the asymmetric shape provides a moment arm or asymmetric portion relative to a longitudinal axis of the catheter shaft to steer the catheter shaft around acute angles, for example though the tricuspid and pulmonary valves for placement in a pulmonary artery. As disclosed, in illustrated embodiments, the delivery lumen is used for intracardiac delivery of materials, imaging catheters or other treatment devices.

Embodiments of the multiple lumen delivery catheter include an inner balloon in the delivery lumen to engage an imaging or echocardiography catheter inserted in the delivery lumen. As disclosed, in illustrated embodiments the length of the delivery catheter is sized for intracardiac placement in a pulmonary artery or other intracardiac site. The delivery catheter can be inserted through a femoral vein into the right atrium through the inferior vena cava or through the superior vena cava via a jugular vein. In illustrated embodiments, the elongate length of the catheter shaft has a varied stiffness or flexibility including a relatively flexible distal portion.

The present application provides a kit for intracardiac imaging including a multiple lumen delivery catheter and intracardiac echocardiography catheter for cardiac imaging and treatment. In illustrated embodiments, the multiple lumens include a delivery lumen, a guide wire lumen and an inflation lumen to inflate a flow assist balloon. In embodiments disclosed the delivery lumen has a diameter of at least 8F, 9F or 10F or between 8F-14F or 9F-14F for insertion of the intracardiac echocardiography catheter. For use, the delivery catheter is inserted into a right atrium, right ventricle for placement into a pulmonary artery. The delivery catheter is tracked to the right atrium through the inferior vena cava or superior vena cava.

In alternate embodiments, the delivery catheter is used for placement of an imaging catheter in the superior vena cava, pulmonary artery or alternate intracardiac imaging site. In illustrated embodiments, the catheter shaft includes a guidewire lumen for intracardiac placement, a flow assist balloon and a delivery lumen sized for insertion of an intracardiac echocardiography catheter. In another embodiment, the delivery catheter includes a flow assist balloon and a delivery lumen having a smaller dimension for medication or other treatment or imaging devices or asymmetric balloon and delivery lumen. Details of the present invention are described in the following drawings and description.

### BRIEF DESCRIPTION OF DRAWINGS

FIG. 1A illustrates an embodiment of an assembly of the present application.
FIG. 1B is a cross-sectional view as taken along line 1B-1B of FIG. 1A of the flow assisted delivery catheter shown without a guidewire and imaging catheter inserted.
FIG. 1C illustrates a hemostatic connector coupled to a delivery lumen.
FIG. 1D is a detailed cross-sectional view of a portion of the catheter of FIG. 1A with a flow assist balloon illustrated in a deflated condition.
FIG. 1E is a detailed illustration of a distal end of the assembly of FIG. 1A illustrating the flow assist balloon inflated for use.
FIG. 1F schematically illustrates an embodiment of an intracardiac imaging catheter (ICE) of the present application.
FIG. 1G illustrates a proximal end of the assembly and imaging catheter.
FIGS. 2A-2E progressively illustrate a process for using the assembly of the present application for intracardiac imaging.
FIGS. 3A-3B illustrate another embodiment of the assembly of the present application.
FIG. 4A illustrates another embodiment of the assembly of the present application.
FIG. 4B is a cross-sectional view as taken along line 4B-4B of FIG. 4A.
FIG. 4C is a cross-sectional view of a portion of the delivery catheter shown with the imaging catheter in the delivery lumen and an inner balloon inflated.
FIG. 5A illustrates another embodiment of the assembly of the present application.
FIG. 5B is a cross-sectional view as taken along line 5B-5B of FIG. 5A.
FIG. 5C is a sectional view of the distal end of the delivery catheter shown with a guidewire extending through a guidewire lumen formed along a distal end of the delivery lumen.
FIG. 5D is a cross-sectional view as taken along line 5D-5D of FIG. 5C.
FIG. 5E is a sectional view of the delivery catheter having an ICE catheter inserted into the delivery lumen and the inner balloon inflated to abut the ICE catheter.
FIG. 5F is a cross-sectional view as taken along line 5F-5F of FIG. 5E.
FIGS. 6A-6D progressively illustrate process steps illustrating use of the assembly of the present application.
FIG. 6E is a flow chart illustrating steps for cardiac imaging using a delivery catheter of the present application.
FIG. 7A illustrates another embodiment of an intracardiac delivery catheter of the present application.
FIG, 7B illustrates an end view of a flow assist balloon of an embodiment of the intracardiac delivery catheter.
FIG. 7C is a longitudinal cross-sectional view of the intracardiac delivery catheter of FIG. 7A including a stiff proximal length and a relatively flexible distal length.
FIG. 7D is a cross-sectional view taken along line D-D of FIG. 7C.
FIG. 7E is a cross-sectional view taken along line E-E of FIG. 7C.
FIG. 7F is a cross-sectional view taken along line F-F of FIG. 7C.
FIG. 7G is a cross-sectional view taken along line G-G of FIG. 7C.
FIG. 7H is a detailed view of a distal portion of a shaft of the intracardiac delivery catheter of an illustrative embodiment.
FIG. 7I illustrates an embodiment of an asymmetrical balloon at a distal end of the catheter shaft of the intracardiac delivery catheter.
FIG. 7J schematically illustrates a distal end of the catheter shaft of the embodiment shown in FIGS. 7A-7I with an intracardiac imaging catheter (ICE) extending through a distal opening of the catheter shaft.
FIG. 7K illustrates a proximal end of the intracardiac delivery catheter and assembly.
FIG. 8A schematically illustrates force(s) and moment interacting with an asymmetric balloon.
FIG. 8B schematically illustrates an embodiment for intracardiac navigation of an asymmetric balloon.
FIG. 9A schematically illustrates another embodiment of an intracardiac delivery catheter of the present application.
FIG. 9B illustrates an end view of an asymmetric flow assist balloon.
FIG. 9C is a cross-sectional view taken along line C-C of FIG. 9A.
FIG. 9D is a cross-sectional view taken along line D-D of FIG. 9A.
FIG. 10A illustrates another embodiment of an intracardiac delivery catheter of the present application having a shortened guide wire lumen.
FIG. 10B is an end view of a flow assist balloon of the intracardiac delivery catheter of FIG. 10A.
FIG. 10C is a cross-sectional view of the delivery catheter of FIG. 10A illustrating a shortened guide wire lumen.
FIG. 10D is a cross-sectional view taken along line D-D of FIG. 10C.
FIG. 10E is a cross-sectional view taken along line E-E of FIG. 10C.
FIG. 11A illustrates another embodiment of an intracardiac delivery catheter having a shorted imaging catheter (ICE) lumen.
FIG. 11B is a cross-sectional view of the delivery catheter of FIG. 11A illustrating the imaging catheter lumen.
FIG. 11C is a cross-sectional view taken along line C-C of FIG. 11B.
FIG. 11D is a cross-sectional view taken along line D-D of FIG. 11B.
FIG. 12A illustrates fabrication steps for embodiments of the present application.
FIG. 12B is a flow chart illustrating a fabrication embodiment of a multiple lumen intracardiac delivery catheter of the present application.
FIG. 13 schematically illustrates an embodiment of a steerable distal tip for embodiments of the delivery catheter of the present application.

In the above-referenced FIGS., like numbers are used to identify like parts. It should be noted that features in the FIGS, are not necessarily drawn to scale.

### DETAILED DESCRIPTION OF ILLUSTRATIVE EMBODIMENTS

The present application relates to an assembly for intracardiac imaging. Illustrative embodiments of the assembly use an echocardiography catheter to provide an intracardiac image for medical treatment. Placement of the imaging catheter at an imaging site can be difficult due to the anatomy of the heart or desired image. Embodiments of the present application provide an assembly for placement of the imaging catheter in a pulmonary artery, superior vena cava or other intracardiac site to provide a feedback image for treatment.

Illustrative embodiments of the present assembly include an intracardiac delivery catheter (IDC) 100 for placement of the imaging catheter such as an intracardiac echocardiography catheter (ICE catheter) 102. The intracardiac delivery catheter 100 is formed of an elongate shaft 104 having an elongate length extending between a proximal end 106 and a distal end 108. In an illustrated embodiment, the shaft 104 is formed of a flexible material having sufficient length to track from an insertion site into a pulmonary artery through a patient's right atrium and right ventricle or other treatment as will be described herein. In illustrated embodiments, the insertion site is a femoral vein or jugular vein.

The shaft 104 of the catheter 102 is bendable and has sufficient stiffness and torquability to navigate the circuitous path from the insertion site for intracardiac placement at the imaging site. The length of the catheter body 104 can have varied flexibility to navigate through a patient's body to the intracardiac treatment site. In an illustrated embodiment, the approximate length of the delivery catheter is between 60-90cm, however in other embodiments, the length is between 50-120cm. As shown the catheter 102 includes a flow assist balloon 110 coupled to the catheter shaft 104 proximate to the distal end 108. Inflation fluid is injected into the flow assist balloon 110 through an inflation syringe 112 or other inflation device coupled to the proximal end 106 of the shaft 104. The proximal end 106 of the shaft 104 also includes a hemostatic connector 116 for the ICE catheter 102 and connector 118 providing a port for a guidewire 120. In the illustrated embodiment, the hemostatic connector 116 includes a flushing port 122. As shown, sleeve 124 encloses attachment of the proximal connectors 116, 118 and syringe 112 to form a proximal hub of the delivery catheter 100.

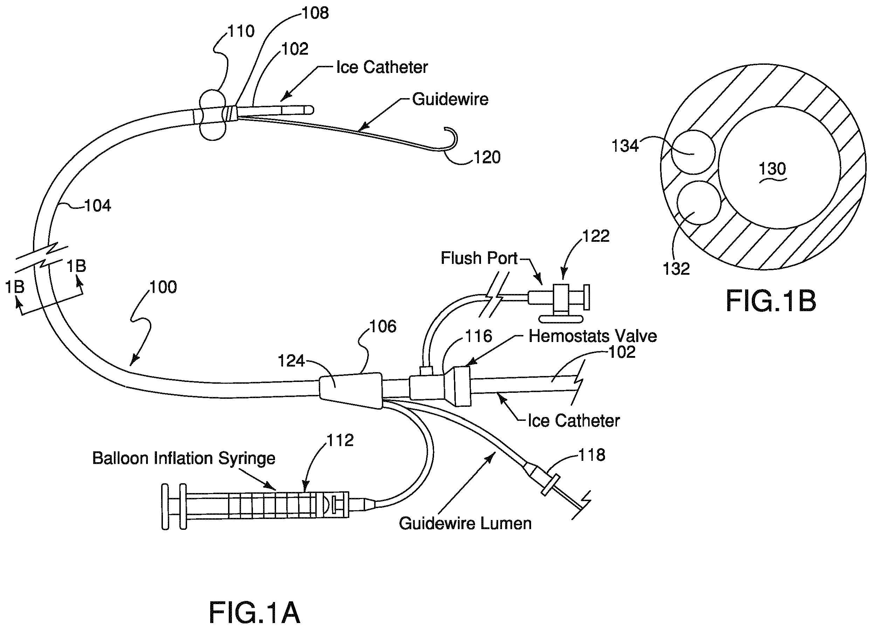

As shown in the cross-sectional view of FIG. 1B, the shaft 104 of the flow assisted delivery catheter 100 includes a plurality of lumens along the length of the shaft 104. The plurality of lumens include an delivery lumen 130 having a diameter sized for insertion of the ICE catheter 102 therethrough, a guidewire lumen 132 and an inflation lumen 134. The delivery catheter lumen 130 has an elongate length that extends from the proximal end 106 of the shaft 104 proximate to the distal end 108. The ICE catheter 102 is inserted through the hemostatic connector 116 coupled to the proximal end of lumen 130 and is slidably advanced through the lumen 130. In the illustrated embodiment, lumen 130 has an opened distal end. In an illustrated embodiment, a diameter of the delivery lumen 130 is at least 8F, 9F or 10F or between 8-14F or 9-14F (French catheter diameter scale) to accommodate a 3D intracardiac echocardiography catheter 102.

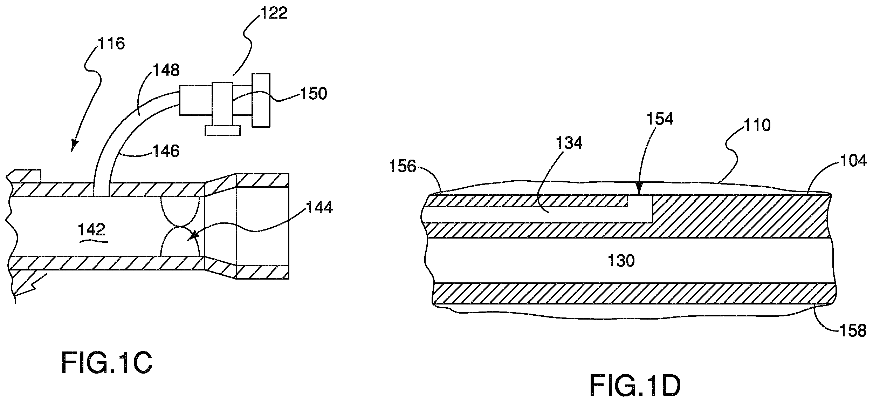

As shown in FIG. 1C, the hemostatic connector 116 is formed of a connector body 140 having a passageway 142 extending between opposed ends of the connector body 140. The connector body 140 includes a flexible hemostatic valve structure (e g. gasket) 144 (illustrated diagrammatically) along the passageway 142. The flushing port 122 is coupled to the passageway 142 of connector 116 through a branch structure 146 having a branch passageway 148. Flow of fluid through the branch passageway 148 is controlled via a flushing port valve 150 operable between an opened position and a closed position to open and close the flushing port 122. Application is not limited to a particular hemostatic valve structure 144 and various designs can be employed as will be appreciated by those skilled in the art.

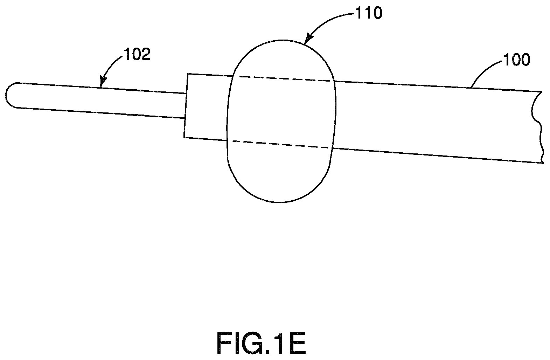

The guidewire lumen 120 has an elongate length that extends from the proximal end 106 of the catheter shaft 104 to the distal end of the catheter shaft 104. The guidewire 120 is inserted through connector 118 and slidably advanced through lumen 132 for placement through an opened distal end of the guidewire lumen 132. The inflation lumen 134 extends along the elongate shaft 104 to inject inflation fluid into the flow assist balloon 110 through an inflation port 154 connecting the inflation lumen 134 to an interior of the flow assist balloon 110 as shown in FIG. 1D. The balloon 110 is formed of an elastomeric or other balloon material having a proximal collar 156 connected about the cylindrical body 104 aft of the inflation portion 154 and a distal collar 158 connected about the cylindrical body 104 forward of the inflation port 154. Inflation fluid is injected into the lumen 134 through syringe 112 to inflate the flow assist delivery balloon 110 as shown in FIG. 1E. As will be appreciated by those skilled in the art, the balloon is a Swan- Ganz type balloon having a construction as described in U.S. Patent No. 3,634,924.

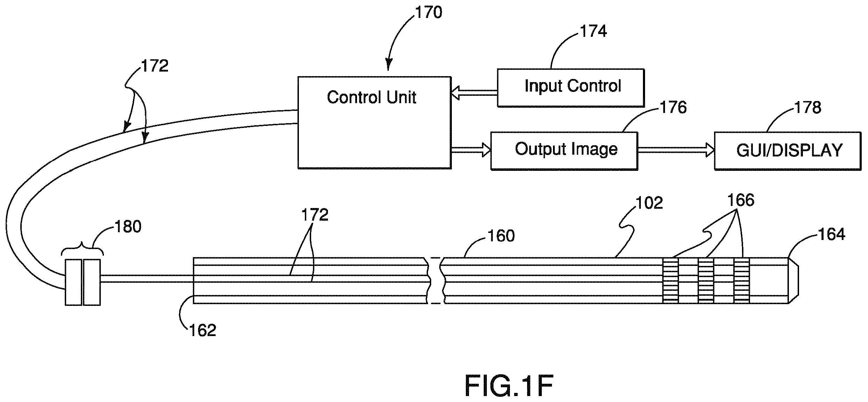

As previously described, the delivery catheter 100 is configured for placement of ICE catheter 102 at an imaging site via insertion through the delivery lumen 130. Illustrative imaging catheters include 2D and 3D intracardiac echocardiography imaging catheters. FIG. 1F is an illustrative embodiment of an ICE imaging catheter 102 for placement via delivery catheter 100. As shown, the ICE catheter 102 has an elongate tubular body or shaft 160 having an elongate length extending between a proximal end 162 and a distal end 164. The elongate length of the tubular body has sufficient flexibility, steerability and length for intracardiac placement in a pulmonary artery or other intracardiac imaging site from an insertion site. A plurality of transducer elements or transducer arrays 166 (illustrated diagrammatically) are coupled to the distal end of the elongate body 160 to generate ultrasonic waves or vibration and detect feedback vibration or excitation for imaging. As shown the transducer elements 166 are coupled to a control unit 170 through a plurality of wires/leads 172 and socket connection 180 to provide an input excitation or vibration and receive feedback vibration or excitation. In an illustrated embodiment, the ICE catheter 102 includes separate transducers 166 for transmitting and receiving ultrasonic vibration or the same transducers can be used as both transmitters and receivers.

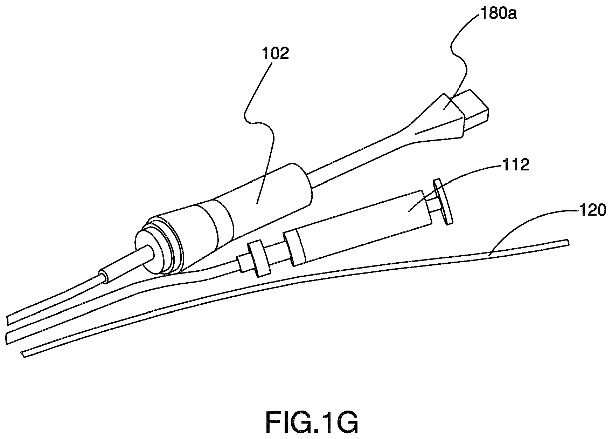

The control unit 170 includes circuitry to generate ultrasonic or echoing high frequency vibration in response to input control 174 and process feedback signals from the transducer elements 166 to provide an output image 176 for display on a GUI device 178. The control unit 170 is coupled to a power source and is coupled to the transducer elements 166 through one or more circuit elements and/or flex circuits. Illustrative transducer elements 166 include an array of piezoelectric elements, capacitive elements or other electromechanical transducer elements for generating ultrasonic vibration or disturbance and detecting feedback vibrations. In an illustrated embodiment as shown in FIGS. 1E-1F, the imaging catheter 102 has a rounded or contoured distal tip for intracardiac placement. As shown in FIG. 1G, in an illustrated embodiment, the plug and socket connection 180 includes a plug 180a connected to the distal end of the ICE catheter 102 which connects to a socket (not shown) connected to the control unit 170.

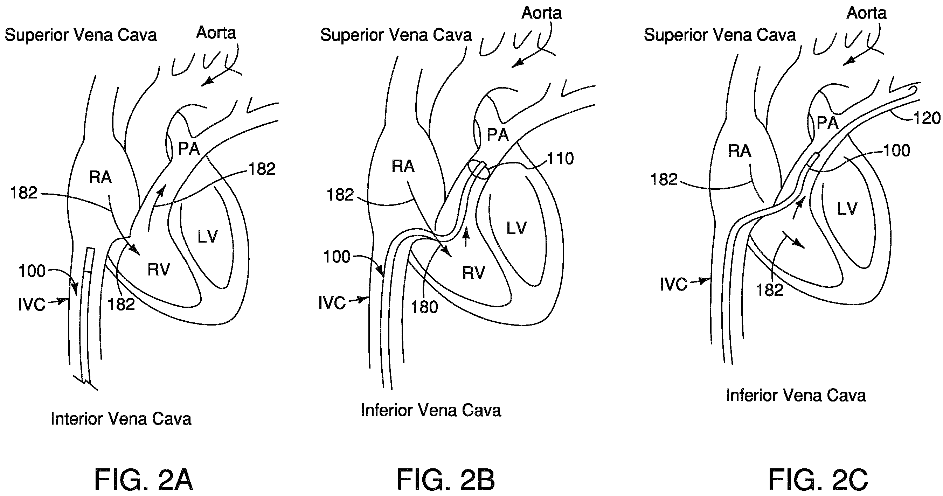

As previously described, the present delivery catheter 100 has application for intracardiac placement of the ICE catheter 102 in the heart. The heart includes a right ventricle (RV) which provides blood flow into the pulmonary artery (PA). Blood flows into the right ventricle (RV) from the right atrium (RA) and from the superior vena cava or the inferior vena cava. The pulmonary artery (PA) is near the aorta which receives blood flow from the right ventricle (RV). The position of the pulmonary artery (PA) near the aorta provides a practical site to image the aorta or other structures of the heart for both electrophysiology and structural procedures. Because of the structure of the heart, placement of the ICE catheter 102 through the right atrium (RA) into the right ventricle (RV) and pulmonary artery (PA) is difficult. In particular, in order to position the imaging catheter 102 into the pulmonary artery (PA) from the right ventricle (RV) the catheter must be guided and steered along an "S" curve path and often can fall back into the right ventricle due to the tortuous path. Use of the present assembly facilitates placement of an ICE catheter 102 as progressively illustrated in FIGS. 2A-2E.

As shown, in FIG. 2A, the delivery catheter 100 is inserted into the right atrium (RA) for example through the inferior vena cava via a femoral vein or the superior vena cava (not shown) through a jugular vein. As shown in FIG. 2B, inflation fluid is injected into the flow assist balloon 110 via inflation lumen 134 to inflate the flow assist balloon 110. The balloon 110 is inflated to take advantage of the blood flow through into and through the right atrium (RA) into the right ventricle (RV) and pulmonary artery (PA). In particular, inflation of the flow assist balloon 110 provides an expanded profile to interface with the blood flow from the right atrium (RA) into the right ventricle (RV) and pulmonary artery (PA) as illustrated by arrows 182. The interface with the blood flow provides an input force or momentum that assists movement or rotation of the delivery catheter 100 from the right atrium (RA) into the right ventricle (RV) and pulmonary artery (PA). In an illustrative embodiment, the balloon is inflated prior to insertion through the tricuspid valve and pulmonary valve to protect the valves from damage.

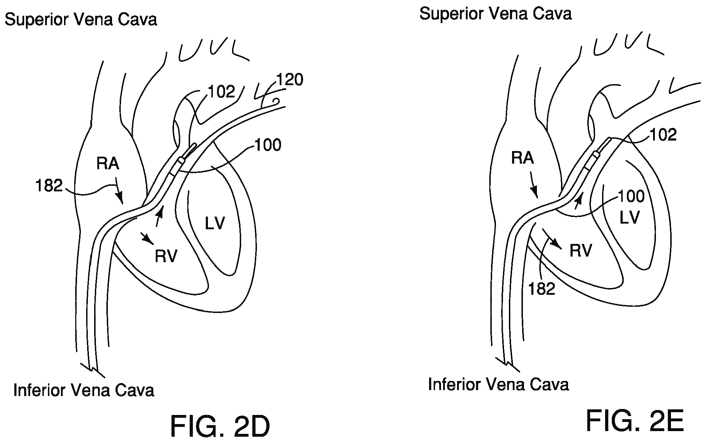

As shown in FIG. 2C, a guidewire 120 is inserted through the guidewire lumen 132 of the delivery catheter 100. The guidewire 120 has sufficient stiffness and rigidity to straighten out the curve from the right atrium (RA) to the pulmonary artery (PA) for preparation for insertion of the intracardiac imaging catheter 102 as shown in FIG. 2D. In particular, the guidewire 120 reduces an "S" curve configuration to provide a straighter path for placement of the ICE catheter 102. As shown in FIG. 2D, the intracardiac imaging catheter 102 is inserted through lumen 130 of the delivery catheter 100 for placement in the pulmonary artery (PA) for imaging. In illustrative embodiments, the guidewire 120 is inserted with the delivery catheter 100 or subsequent to intracardiac placement of the delivery catheter 100. In particular, the delivery catheter 100 can be inserted with the guidewire 120 for support.

As shown in FIG. 2E, once the imaging catheter 102 is in place, the delivery catheter 100 and guidewire 120 are withdrawn. In illustrative embodiments, the guide wire 120 is useful to readvanced the delivery catheter 100 or other catheter into the pulmonary artery from the right ventricle (RV) or superior vena cava from the right atrium for treatment or imaging and is retained in the guidewire lumen 132. In alternate embodiments, the guidewire 120 is withdrawn as shown in FIG. 2E, but the delivery catheter 100 remains in place in the pulmonary artery (PA) during imaging or both the delivery catheter 100 and guidewire 120 remain in place.

In particular, in an illustrated embodiment, the imaging catheter 102 and transducer elements 166 are disposed in the delivery lumen 130 during imaging as described in illustrated embodiments herein. A transmission fluid is inserted into the delivery lumen through port 122 to provide a transmission medium for imaging. In alternate embodiments, the length of the delivery catheter 100 is sized so that the ICE or imaging catheter 102 extends outside the delivery catheter lumen 130 during imaging. In illustrative embodiments, the delivery catheter 100 remains in place to prevent prolapse of the ICE catheter 102 and facilitate torquability of the ICE catheter 102. The delivery catheter 100 can prevent prolapse of the imaging ICE catheter 102 from the (PA) into the (RV) and from the superior vena cava (SVC) into (RA).

Thus, as described, the delivery catheter 100 facilitates intracardiac placement of ICE catheter 102. In one embodiment, the ICE catheter 102 is positioned in the mid right atrium (RA) and the transducer array 166 or transducers are orientated toward the tricuspid valve to image the right ventricle, a portion of the right atrium and the tricuspid valve. In another embodiment, the ICE catheter 102 is orientated to provide an image of the aortic valve, coronary sinus and atrial septum for a transseptal procedure. The ICE catheter 102 can be placed in the right ventricle and orientated to image the left ventricle to screen for effusion or thrombus or mitral valve anatomy. Use of the delivery catheter 100 with the ICE catheter 102 enhances torquability of the ICE catheter 102 within the delivery catheter 100. The delivery catheter may also be positioned across the atrial septum into a pulmonary vein to support an ICE probe to examine the mitral valve or left atrial appendage.

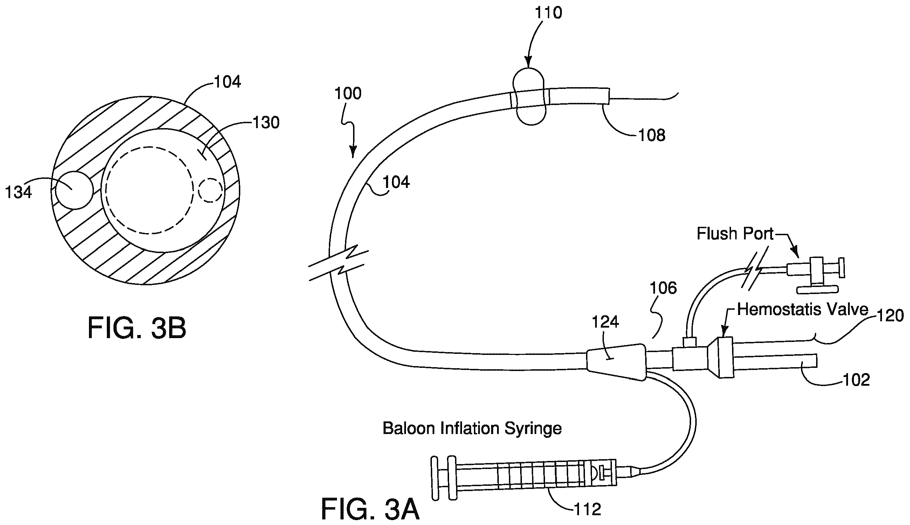

FIGS. 3A-3B illustrate an alternate embodiment of an assembly of the present application including a flow assisted delivery catheter 100 in combination with an imaging or ICE catheter 102 where like numbers are used to refer to like parts in the previous FIGS. As shown in FIGS. 3A-3B, the shaft 104 includes a delivery lumen 130 sized for insertion of the ICE or imaging catheter 102 and guidewire 120 therethrough. The guidewire 120 and ICE catheter 102 are inserted through connector 116 into the delivery lumen 130 for placement of the wire 120 and ICE catheter 102 for treatment as previously described and illustrated in FIGS. 2A- 2E. The flow assist balloon 110 is inflated via fluid injected into the inflation lumen 134 via syringe 112 through inflation port 154 as described in the previous embodiment. In an alternate embodiment not shown, the delivery lumen 130 is sized to accommodate insertion of the ICE catheter 102 and guidewire 120 individually and the guidewire 120 is withdrawn prior to insertion of the ICE catheter 102 as described in illustrative embodiments.

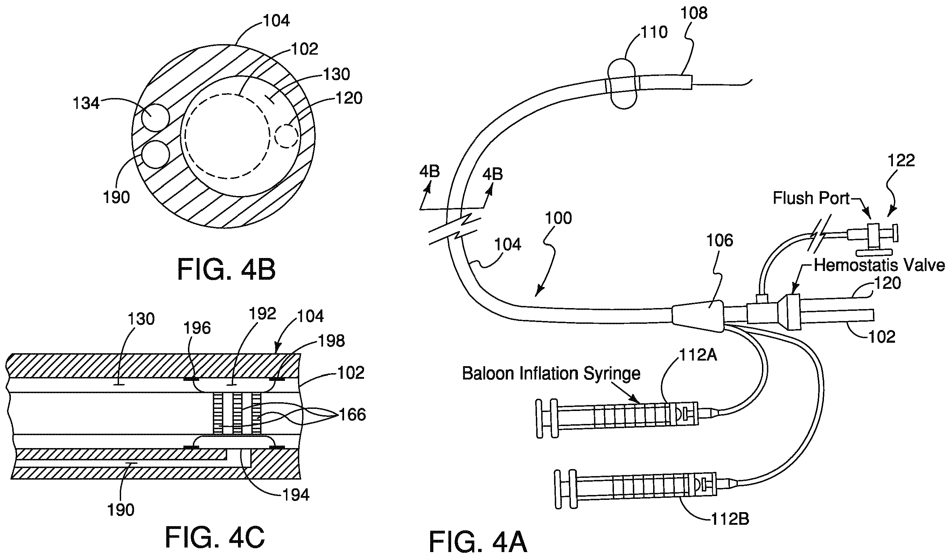

FIGS. 4A-4B illustrate another embodiment of the delivery catheter 100 including a flow assist balloon 110 coupled to the distal end of the delivery catheter 100 and plurality of lumens along the body 104 as shown in FIG. 4B. The plurality of lumens include a delivery lumen 130 which is sized to accommodate the guidewire 120 and ICE catheter as shown in FIG. 4A and inflation lumen 134 to inflate the flow assist delivery balloon 110. The flow assist balloon 110 is inflated via inflation fluid injected into inflation lumen 134 through syringe 112A as shown in FIG. 4A. The delivery catheter 100 as shown in FIG. 4B also includes an inflation lumen 190 to inflate an inner balloon 192 disposed in the distal end of the delivery lumen 130 as shown in FIG. 4C. Inflation fluid is injected through the lumen 190 into the balloon 192 through inlet port 194.

The inner balloon 192 is inflated to retain the ICE catheter and provide an ultrasonic transmission fluid or medium through the inner balloon 192 as shown in FIG. 4C. In particular, the inner balloon 192 includes a proximal collar 196 coupled to the inner surface of the delivery lumen 130 aft of the inlet port 194 and a distal collar 198 coupled to the delivery lumen 130 forward of the inlet port 194. Inflation fluid is injected into lumen 190 via syringe 112B to inflate the inner balloon 192 to engage the ICE catheter disposed in the delivery lumen 130. In an alternate embodiment not shown, the embodiment shown in FIGS. 4A-4B with the inner balloon 192 includes a separate guidewire lumen 132.

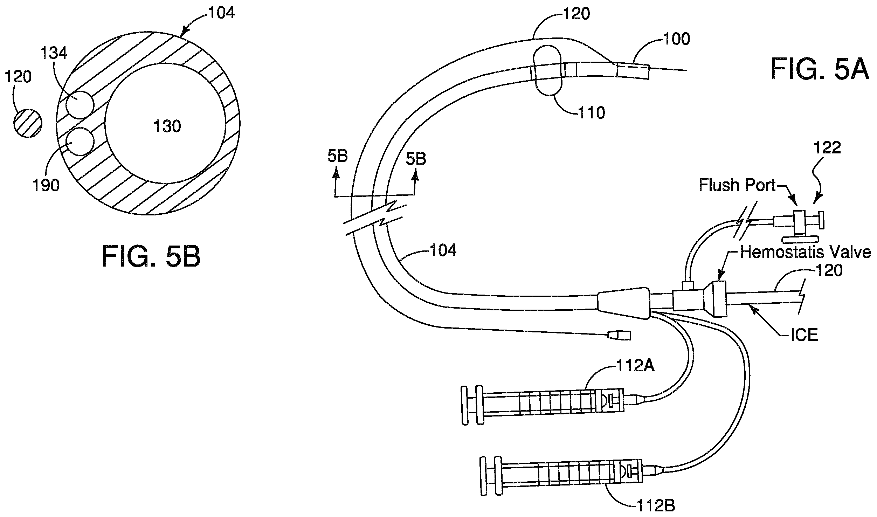

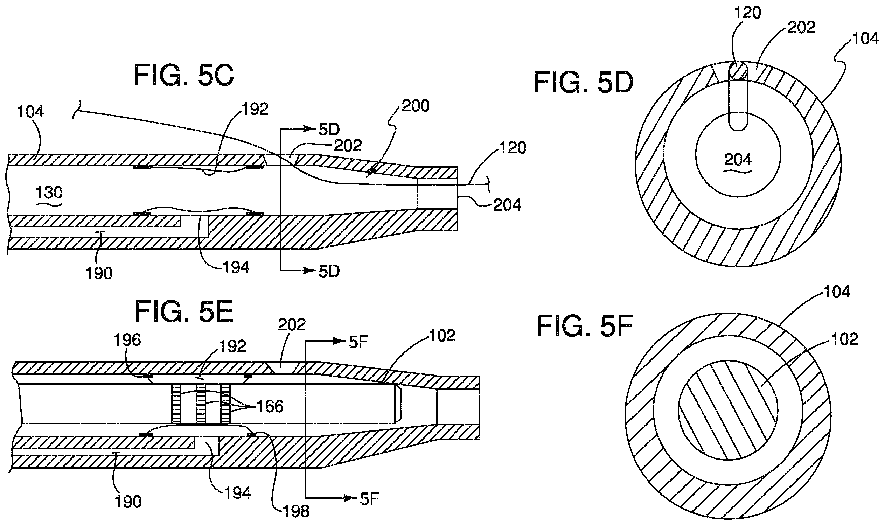

FIGS. 5A-5F illustrate another embodiment of an assembly of the present application. As shown, the assembly includes a delivery catheter 100 having an elongate shaft 104 and elongate guidewire 120. The delivery catheter 100 includes a flow assist balloon 110 coupled to the distal end of the elongate shaft 104 for intracardiac placement as previously described. The elongate shaft 104 includes a plurality of lumens including a delivery lumen 130 for an ICE catheter, inflation lumen 134 for inflating the flow assist balloon 110 and an inner inflation lumen 190 to inflate an inner balloon 192. Fluid is supplied into the inflation lumen 134 via syringe 112A and into inflation lumen 190 through syringe 112B.

As shown in FIGS. 5A and 5C the delivery catheter includes a rapid exchange guidewire lumen 200 at the distal end of the delivery catheter 100 for the guidewire 120. In the illustrated embodiment, the rapid exchange guidewire lumen 200 is formed along a distal length of the delivery lumen 130. The rapid exchange guidewire lumen 200 includes a proximal opening 202 formed through the shaft 104 into the delivery lumen 130 and a distal opening 204 formed through an opened distal end of the delivery lumen 130. The distal end of the wire 120 extends through the distal opening 204 along the delivery lumen 130 (guidewire lumen 200) and through the proximal opening 202 to track the delivery catheter 100 to the treatment site as shown in FIGS. 5C-5D.

As shown in FIG. 5E, the delivery catheter 100 also includes inner balloon 192 along the delivery lumen 130. The inner balloon 192 is inflated to engage the ICE catheter 102 inserted through delivery lumen 130 as shown in FIG. 5E. Fluid is injected into the inner balloon 192 through the inner inflation lumen 190 to inflate the inner balloon 192 via syringe 112B to abut the ICE catheter 102. As shown the inner balloon 192 includes proximal collar 196 coupled to inner surface of the delivery lumen 130 aft of the inlet port 194 and distal collar 196 coupled to an inner surface of the delivery lumen 130 spaced forward of the inlet 194. The inflated inner balloon 192 stabilizes the ICE catheter 102 and provides a transmission fluid surrounding the ICE catheter and in particular, the transducer elements 166 of the ICE catheter.

While in the illustrated embodiments, the rapid exchange or shortened guidewire lumen 200 is formed along a distal length of the delivery lumen 130, application is not limited to the illustrated embodiment and the rapid exchange guidewire lumen 200 can be formed of a separate shorted guidewire lumen at the distal end of the delivery catheter 100. Further the delivery catheter 100 can include a plurality of openings 202 for the guide wire 120 spaced about the circumference of the delivery catheter 100. In an illustrative embodiment, opening 202 is spaced 1-3 centimeters from the distal tip or end 108 of the delivery catheter 100.

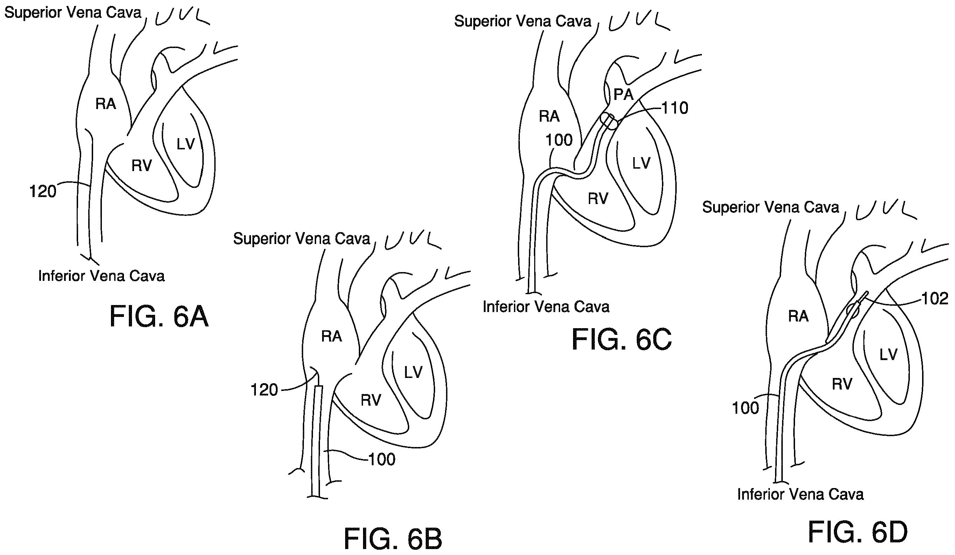

FIGS. 6A-6D illustrate use of delivery catheter 100 having a rapid exchange or shortened guidewire lumen 200 as described. As shown, in FIG. 6A, in the illustrated embodiment, the guidewire 120 is percutaneously inserted into the patient and guided for intracardiac placement. In particular, in the illustrated embodiment, the guidewire 120 is advanced intravascularly into the right atrium to facilitate insertion of the delivery catheter 100. As shown, in FIG. 6B, the delivery catheter 100 is advanced along the guidewire 120 for intracardiac placement. As shown in FIG. 6C, inflation fluid is injected through syringe 112A to inflate balloon 110 to provide flow assistance or force to track the delivery catheter 100 into the right ventricle and the pulmonary artery to a treatment or imaging site. In particular, the balloon 110 is inflated to use blood flow to track the delivery catheter 100 into the right atrium, right ventricle and pulmonary artery.

In FIG. 6D when the delivery catheter 100 is in place, the balloon 110 is deflated and the ICE catheter 102 is inserted. In illustrative embodiments, the balloon 110 is deflated and the guidewire 120 is withdrawn prior to placement of the ICE catheter 102 or in illustrated embodiments the guidewire is withdrawn prior to inflation of the flow assist balloon 110. In other embodiments, the guidewire remains in place for insertion of the imaging catheter 102 and during imaging. As previously described, the delivery catheter 100 can be withdrawn or remain in place during imaging.

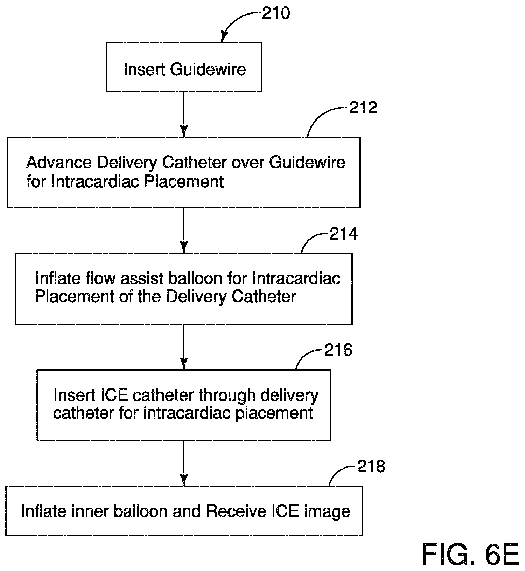

FIG. 6E illustrates steps of the illustrated assemblies of the present application. As shown in step 210, a guidewire 120 is inserted and tracked intravascularly for intracardiac placement, for example to insert the delivery catheter 100 into the right atrium. In step 212, the delivery catheter 100 is inserted for intracardiac placement using the guidewire 120. In step 214 the flow assist balloon 110 on the delivery catheter 110 is inflated to assist placement of the delivery catheter 100 at the intracardiac treatment site, for example into the right atrium, right ventricle or pulmonary artery. In step 216 the ICE or imaging catheter 102 is advanced through the delivery lumen 130 to an imaging or treatment site for example, in the pulmonary artery. In an example embodiment, the flow assist balloon 110 is deflated prior to insertion of the ICE catheter 102. The inner balloon 190 is inflated in step 218 to abut the ICE catheter 102 and provide a fluid bladder for transmission of ultrasonic imaging waves during use. Alternatively, fluid is injected into the delivery lumen for imaging. In illustrative embodiments, the guidewire is withdrawn prior to insertion of the ICE catheter or in an alternate embodiment the guidewire 120 is withdrawn prior to inflation of the flow assist balloon. In illustrative embodiments, the delivery catheter 100 includes a separate guidewire lumen 132,200 and the guidewire 120 remains in place to reposition the delivery catheter 100.

In an alternate embodiment of a delivery catheter 100 having an inner balloon 192 and outer balloon 110, the delivery catheter 100 includes delivery lumen 130, inflation lumen 134, inflation lumen 190 and a guide wire lumen 120 that extends along the length of the delivery catheter between the proximal and distal ends.

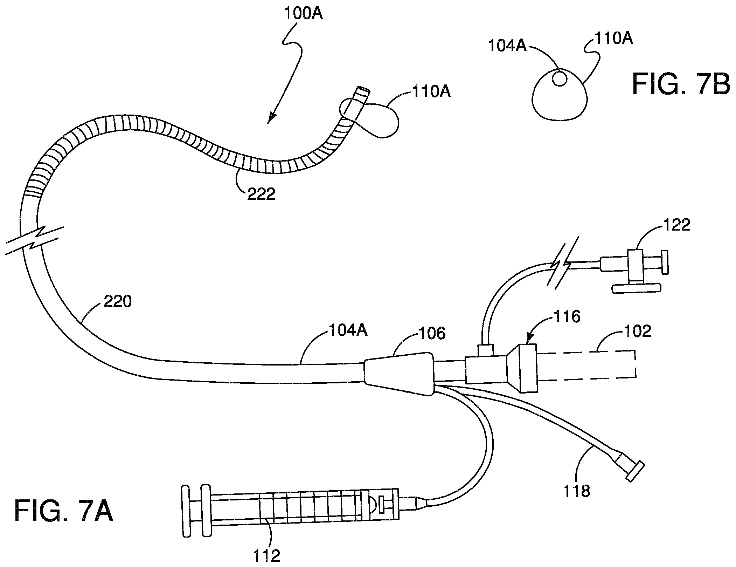

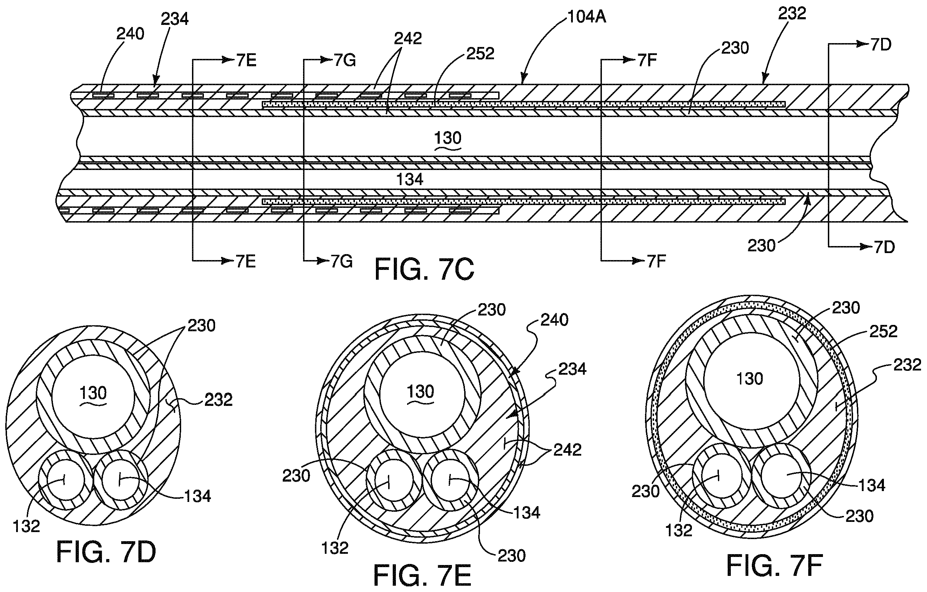

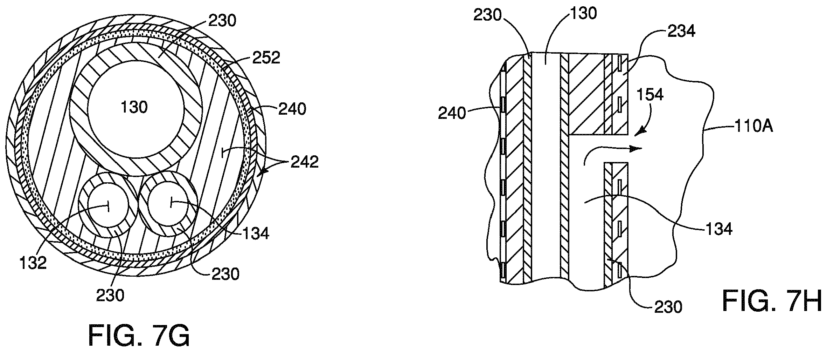

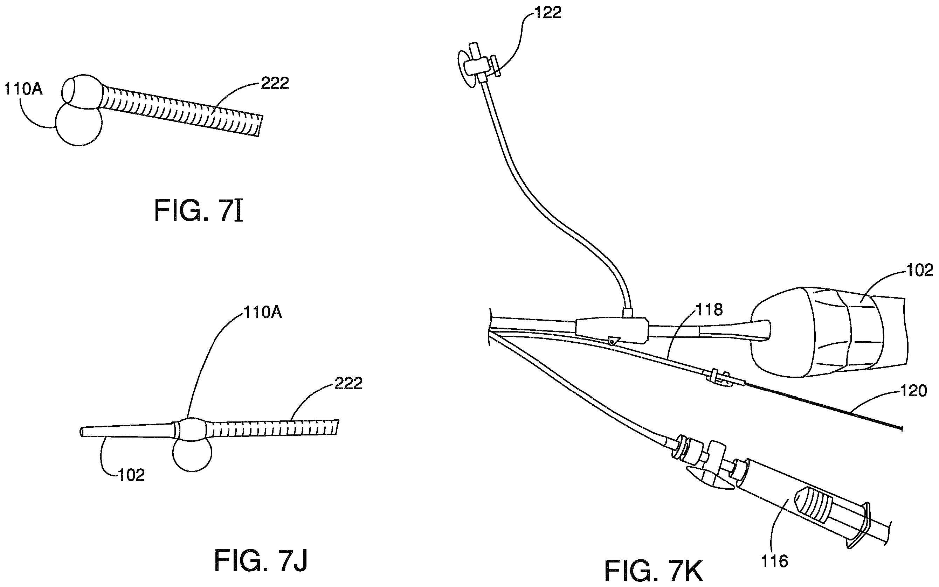

FIG. 7A illustrates another embodiment of an intracardiac echocardiography delivery catheter 100A having an elongate cylindrical shaped body or shaft 104A where like numbers are used to identify like parts in the previous FIGS. As shown, the elongate body or shaft 104A includes a relatively stiff proximal length 220 and a relatively or ultra-flexible distal length 222. The relatively stiff proximal length 220 provides pushability and torqueability for insertion and the relatively flexible length 222 provides sufficient flexibility or bendability for intracardiac navigation, for example, through the inferior vena cava into the right atrium, right ventricle and into the pulmonary artery for placement of an imaging or ICE catheter 102. As previously described, placement of the delivery catheter 100A is assisted by a flow assist balloon 110A at the distal end of the catheter shaft 104 A (or ultra-flexible distal length 222).

In the illustrated embodiment, the balloon 110A is an asymmetrical balloon as shown in FIG. 7B to enhance navigation and placement of the catheter 100A as will be described herein. Balloon 110A is inflated via syringe 112 to assist placement of the delivery catheter 100 A as previously described. In an illustrative embodiment, a length dimension of the relatively stiff proximal length 220 of the catheter is approximately 30-50 cm (11-20 inches) and a length dimensions of the flexible distal length 222 of the catheter is approximately, 30 cm (11 inches).

In the illustrated embodiment shown in FIG. 7A, the catheter shaft 104A of the delivery catheter 100A is a multiple lumen shaft 104A as shown in FIGS. 7C-7G. As shown, the multiple lumen shaft 104A includes an ICE delivery lumen 130, guidewire lumen 132, and a balloon inflation lumen 134 formed along the proximal and distal lengths 220, 222 of the shaft 104A. In the embodiment shown, the multiple lumens are formed along an inner core or lumen structure formed of a plurality of microtubes 230 for each of the plurality of lumens. In illustrated embodiments, the plurality of microtubes 230 are formed of polytetraflouralethlene (PTFE), such as PTFE sold under the trademark Teflon^{®} by Chemours Company FC of Delaware. The Teflon^{®} microtubes 230 provide a lubricous inner surface and slideable placement and movement of the ICE catheter 102 and guide wire 120.

The stiff proximal length 220 is formed of a stiff perimeter or wall structure 232 formed about the core or lumen structure. In illustrated embodiments, the stiff perimeter structure 232 of the proximal length 220 is formed of hard durometer polymer material such as a polyether block amide, for example, Pebax^{®} available from Arkema France Corporation of Colombes France having a hardness range of 63 Shore D, or polyvinyl alcohol (PVA1) urethane material or similar material having a hardness range between 25- 72 Shore D to form the relatively stiff proximal length 220 of the catheter shaft 104A.

The flexible distal length 222 of the catheter shaft 104A has a relatively flexible perimeter or wall structure 234 formed of helical coil 240 embedded within a soft polymer body or layers 242 (inner and outer layers). The helical coil 240 is formed of an aluminum, stainless steel or other material. In an illustrated embodiment the helical coil 240 provides a relatively high hoop strength to provide structural integrity for the multiple lumens of the catheter shaft 104 A and the polymer body or layers 242 and embedded helical coil 240 provide a flexible and bendable distal length for intracardiac placement of the catheter 100A. Illustratively, flexible polymer body or layers 242 are formed of a low durometer or hardness material to provide the flexibility for the distal length 222. Illustrative low durometer materials include polychloropene layer(s) or material having a hardness of approximately 60 Shore A or other material having similar durometer or hardness.

As shown in FIGS. 7C the catheter shaft 104A includes a transition structure connecting the proximal and distal lengths 220, 222 to form the elongate length of the catheter shaft 104A. As shown, the transition structure includes a tubular braid 252 having a length that extends along a transition zone between the proximal and distal lengths 220, 222 to connect the proximal and distal lengths of the catheter shaft 104A. The tubular braid 252 encloses the core or lumen structure as shown FIGS. 7C and 7F and is formed of a Nitinol - nickel-titanium alloy, stainless steel or fabric material. As shown, the relatively stiff perimeter structure 232 of the proximal length 220 and flexible perimeter or wall structure 234 of the distal length 222 are formed along the proximal and distal ends of the transition zone, respectively. In addition to the structure shown, in illustrated embodiments, the catheter shaft 104A includes on or more stiffening wires formed along the structure of the catheter shaft 104A for steerability.

In the illustrated embodiment shown, the core or lumen structure is formed of a plurality of microtubes 230 that extend along the length of the catheter shaft 104A, however, in alternate embodiments, the microtubes 230 extend along the proximal length of the catheter shaft and the lumens along the distal length are formed or molded along the distal perimeter structure 234 or shaft 104A. As previously described, and as shown in FIG. 7H, the length of the inflation lumen 134 extends from the proximal end of the catheter shaft 104A to a distal port 154 opened to an interior of the balloon 110A to inflate the balloon 110A at the distal end 108 of the catheter shaft 104A as shown in FIGS. 7I-7J. The guide wire lumen 132 and ICE delivery lumen 130 extend the length of the catheter shaft 104A from the proximal end 106 to distal openings at the end of the catheter shaft 104A. FIG. 7K illustrates the syringe 112 (with control valve), ICE catheter 102 and guide wire connector 118 or port at the proximal end of the catheter shaft 104A.

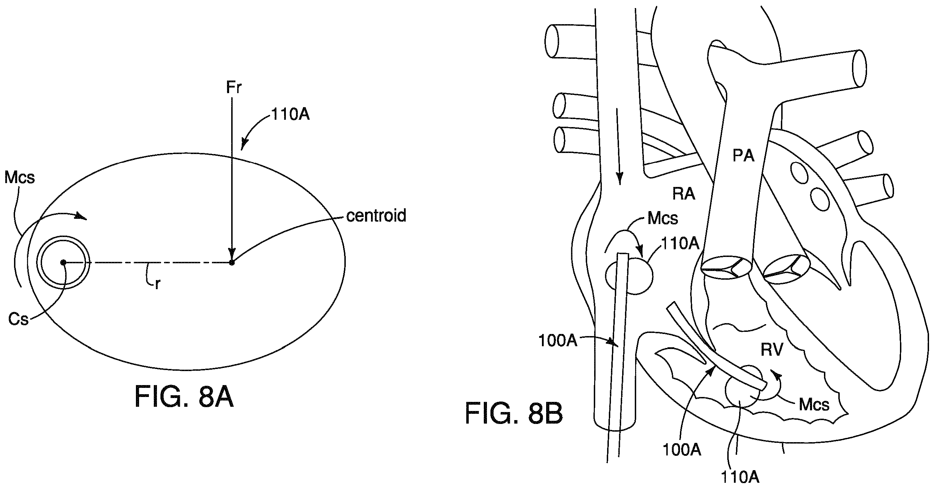

As previously described and shown in FIG. 7B, in an illustrative embodiment, the balloon 110A is an asymmetric balloon where centroid c of the body of the balloon 110A is offset from a longitudinal axis of the catheter shaft Cₛ as shown in FIG. 8A. During use as schematically shown in FIG. 8A, blood flow or cardiac pressure provides a distributed force along a surface of the balloon 110A. In contrast to a symmetrical balloon, force applied to the asymmetrical balloon 110A imparts a resultant force Fᵣ through a centroid c of the balloon spaced from the longitudinal axis of the catheter shaft Cₛ to define a moment arm r or asymmetric portion between the catheter shaft Cs and centroid c. The resultant force Fr and moment arm r imparts a moment M_{cs} relative to the catheter shaft Cₛ having a magnitude provided by M_{cs}= Fᵣ* r. Thus, as described, the asymmetric portion of the balloon 100A is orientated to assist with steering and placement of the delivery catheter 100. For example, the asymmetric portion is oriented to steer the catheter 100 through the tricuspid valve into the right ventricle and through the pulmonary valve into the pulmonary artery.

As shown in FIG. 8B during use and insertion, the balloon is orientated to impart a clockwise moment Mes to align a tip of the delivery catheter 100A with the tricuspid valve for insertion into the (RV) and as shown in FIG. 8B, the balloon 110A is orientated to impart a counterclockwise moment Mes relative to the catheter shaft 104A to align the tip of the delivery catheter 100A with the pulmonary artery (PA) for access to an imaging or treatment site. Additionally, the off-axis alignment of the balloon reduces the surface area to reduce back pressure and force imparted to the catheter 100A during insertion.

While illustrative embodiments show application of the asymmetric balloon for placement of the delivery catheter 100A, application of the asymmetric balloon 110A as described is not limited to delivery catheter 100A and can be used for navigation of other intracardiac or delivery catheters into the body or to a treatment site. In an illustrative embodiment, the asymmetric balloon 110A is formed of an elongate generally tubular shaped body having spaced first and second ends connected to an outer surface of the flexible perimeter or wall structure 234 or catheter shaft 104A. The asymmetric shape is formed via a thinner or stressed wall thickness of the tubular shaped body so that inflation of the interior cavity of the balloon forms the asymmetric shape of the balloon 110A.

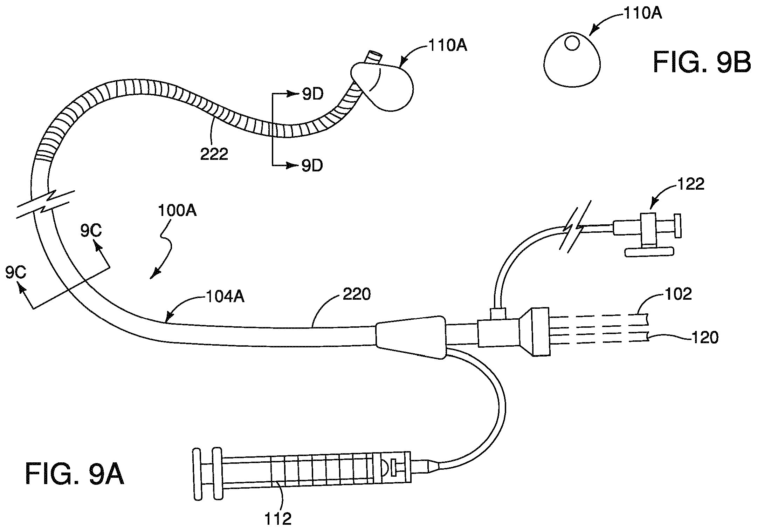

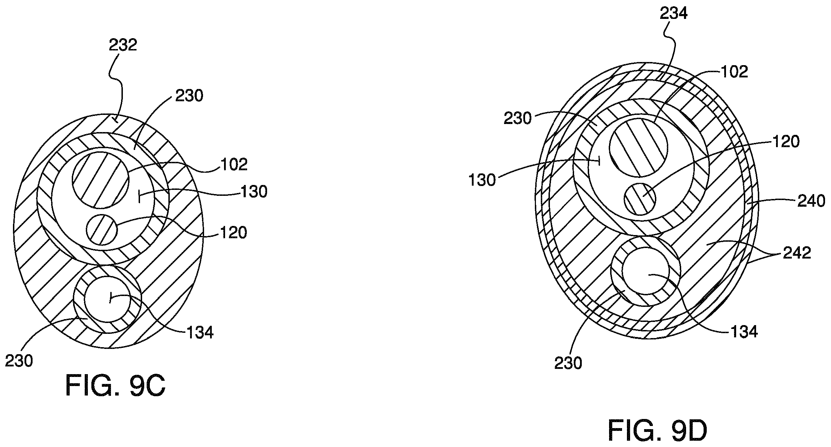

FIGS. 9A- 9D illustrate an alternate embodiment of the delivery catheter 100A having a stiff proximal length 220 and are relatively flexible distal length 222 as previously described with respect to FIGS. 7A-7G. In the embodiment shown, the multiple lumen catheter shaft 104A includes ICE delivery lumen 130 and balloon inflation lumen 134. Similar to the embodiment illustrated in FIGS 3A-3B, the delivery lumen 130 is sized to accommodate the ICE imaging catheter 102 and guidewire 120 for placement of the delivery catheter 100A.

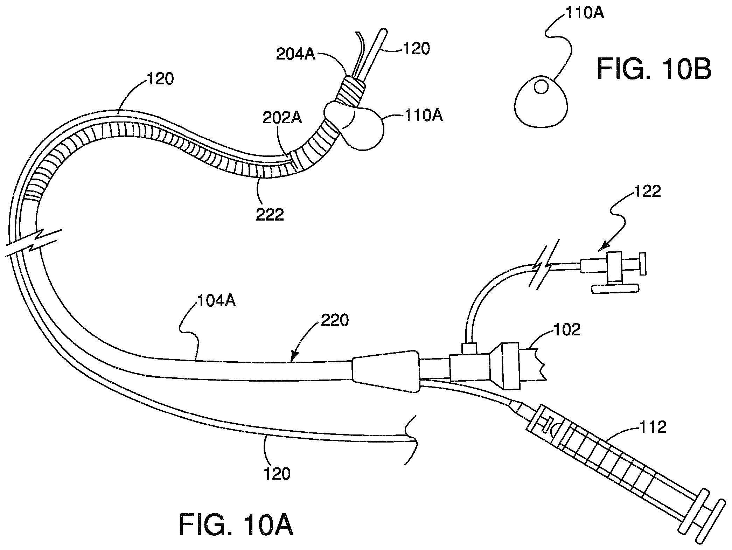

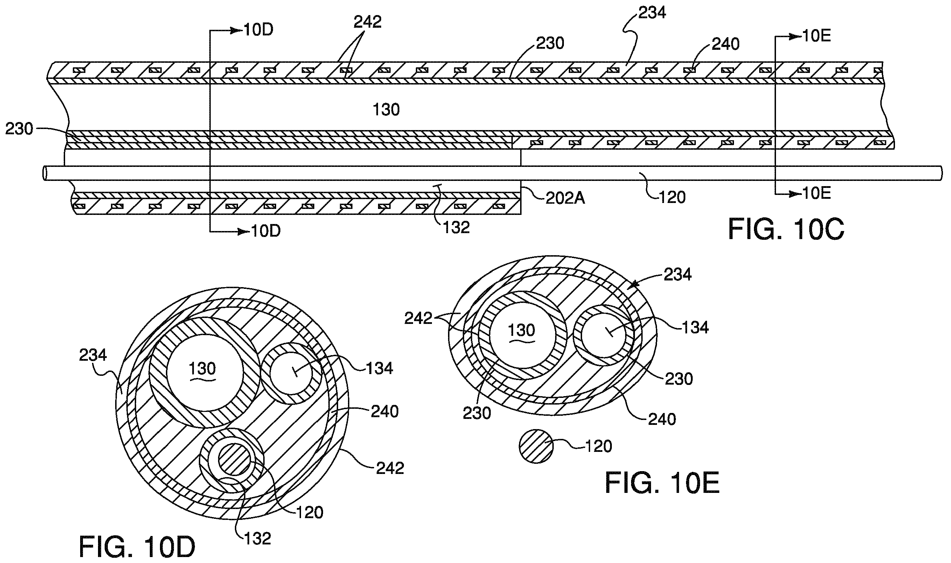

FIGS. 10A-10E illustrate another embodiment of a delivery catheter 100A including a stiff proximal length 220 and a flexible distal length 222 as previously described. In the illustrated embodiment shown, the shaft 104A includes an ICE delivery lumen 130 and an inflation lumen 134 along the proximal and distal lengths 220, 222. As shown in FIG. 10C, the distal length includes a shortened guidewire lumen 132 (200). The rapid exchange or shortened guidewire lumen 132 (200) includes a proximal opening 202A and a distal opening 204A (as shown in FIG. 10A) formed through an opened distal end of lumen 132 at the distal end of the catheter shaft 104A. The distal end of the wire 120 extends through the proximal opening 202A and the distal opening 204A to track the delivery catheter to the treatment site. The proximal opening 202A is spaced from the proximal end of the catheter shaft 104A. In the embodiment shown, the length of the rapid exchange or shortened guidewire lumen 132 (200) extends along the flexible distal length 222, however, application is not limited to the length of the rapid exchange guide wire lumen 132 shown and in illustrative embodiments the length can extend along the entire flexible distal length 222 or along the distal length and a portion of the stiff proximal length 220 so that the proximal opening 202A is located along the stiff proximal length 220.

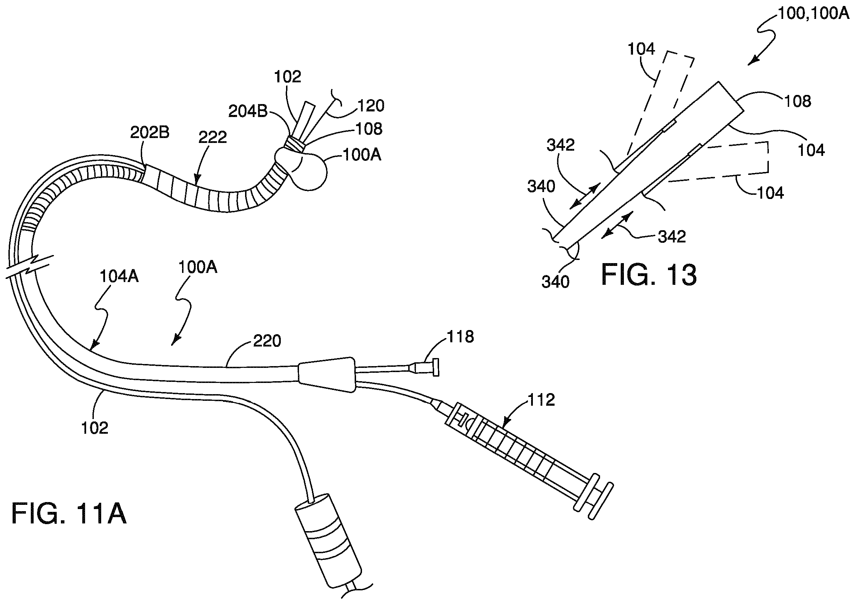

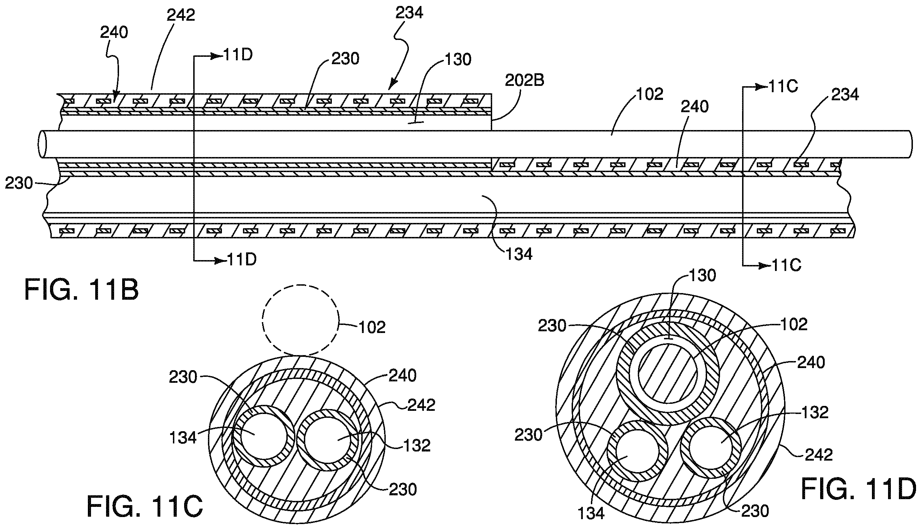

FIGS. 11A-11D illustrate another embodiment of a delivery catheter 100A including a stiff proximal length 220 and a flexible distal length 222 as previously described. In the illustrated embodiment, the catheter 100A includes a shortened ICE delivery lumen 130 along the relatively flexible distal length 222 of the catheter shaft as shown in FIG. 11B. As shown, the flexible distal length 222 includes ICE delivery lumen 130 having a proximal opening 202B spaced from the proximal end 106 of the catheter shaft 104A and a distal opening 204B (shown in FIG. 11A) at the distal end 108 of the catheter shaft 104A.

Thus, as shown, the ICE delivery lumen 130 is formed along the flexible distal length 222 of the catheter shaft. The stiff proximal length 220 provides sufficient rigidity and pushability to insert the delivery catheter 100A and ICE catheter 102 through a patient's vascular to the right atrium. Guide wire 120 is inserted through lumen 132 and the balloon 110A is inflated to float the delivery catheter into right ventricle (RV) and pulmonary artery (PA) for imaging. In alternate embodiment, not shown, both the guide wire lumen 132 and ICE delivery lumen 130 have a shortened length extending along the flexible distal length 222 of the catheter shaft 104A. In an alternate embodiment not shown, the shortened ICE delivery lumen 130 extends along the flexible distal length 222 and a portion of the stiff proximal length 220 so that proximal opening 202B is disposed along the stiff proximal length 220 of the catheter shaft 104A.

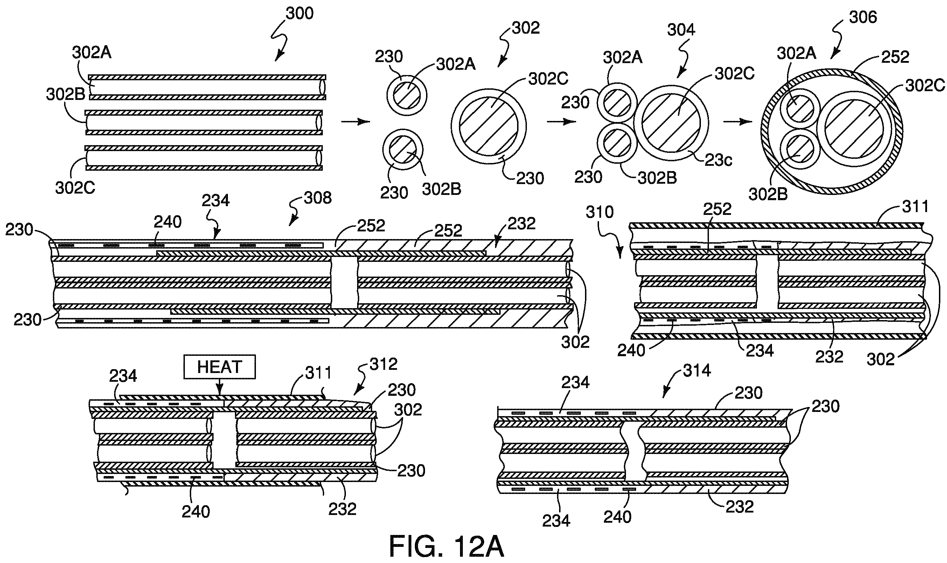

FIG. 12A schematically illustrates fabrication of a multiple lumen delivery catheter 100A having a stiff proximal length 220 and an ultra-flexible distal length 222 in accordance with illustrative embodiments described. As progressively shown in step 300, a plurality of mandrels 302A, 302B, 302C having different sized diameters to correspond to the different sized lumens are coated with a Teflon^{®} coating or other lubricous material. In step 302, microtubes 230 are assembled on the mandrels 302A-302C to form the plurality of lumens. As previously discussed, in illustrated embodiments, the microtubes 230 are formed of a Teflon material to provide a lubricous inner surface of the lumens. In step 304, the microtubes 230 are joined along a length thereof to form a multiple lumen or core structure. Illustratively, the microtubes 230 are connected via an adhesive or other material. An outer surface of the tubular liners can be roughened to enhance the adhesive connection of the microtubes 230.

As shown in step 306, a tubular braid 252 is deposited over an outer circumference of the microtubes to form the transition between the proximal and distal lengths 220, 222 of the catheter shaft. In step 308, an outer surface of the core or lumen structure is coated with the hard polymer material along proximal length and tubular braid 252 along a transition length and the soft polymer material or layers 242 and helical coil 240 are deposited over a distal length of the core or lumen structure and a transition length of the tubular braid 252 to form the proximal and distal lengths 220, 222 of the catheter shaft 104A. As shown in step 310, a heat shrink tube 311 is placed about the length of the structure and in step 312, heat is supplied to shrink tube 311 to impart pressure to the polymer coatings or layers. In an illustrated embodiment the heat shrink tube is a fluorinated ethylene propylene (FEP) heat shrink tube. Heat and pressure imparted by the shrinking diameter of the shrink tube 311 causes the polymer coatings or layers to flow into the interspaces of the tubular braid 252 to connect the relatively stiff proximal length 220 of the catheter and the relatively flexible distal length 222 of the catheter and coil. Thereafter, in step 314 the heat shrink tube 311 and mandrels 302 are removed for assembly of the proximal hub and balloon to the catheter shaft 104 to form the delivery catheter 100A.

In alternate embodiments, the length of the microtubes 230 extends along the proximal length 220 and not along the distal length 222 and the polymer coating or layers form the lumen structure for the multiple lumens along the distal length 222. In alternate embodiments as disclosed shortened microtubes 230 are used to form a shortened distal guide wire lumen 132 or ICE delivery lumen 130.

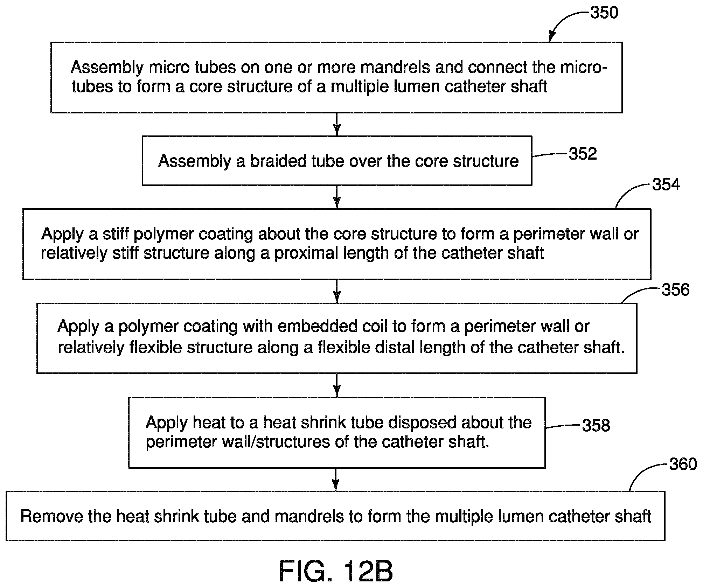

FIG. 12B is a flow chart illustrating process steps for fabrication of the delivery catheter 100A and shaft according to embodiments of the present application. As previously described a plurality of mandrels 302 are used to form a plurality of lumens of a multiple lumen catheter shaft. As shown in step 350 a plurality of microtubes 230 are assembled over mandrels and adhesively connected to form the multiple lumen/core structure. In step 352, tubular braid 252 is assembled over the lumen or core structure to form a transition zone. In step 354 a proximal length of the structure is coated with a hard durometer polymer material to form the stiff proximal length 220 of the catheter. In step 356, a helical spring is assembled over the distal length between inner and outer low durometer polymer layers to form the flexible distal length 222. In step 358 heat is applied to a heat shrink tube 311 to provide heat and pressure about an outer circumference of the coatings or layers to provide material flow to form the perimeter wall structures of the proximal and distal lengths 220, 222 of the catheter shaft connected along a transition length through the braided tube 252. In step 360 the heat shrink tube 311 and mandrels 302 are removed.

FIG. 13 illustrates a distal tip of an illustrative embodiments of the delivery catheter 100, 100A. As shown, elongate control wires 340 are coupled to the distal tip of the delivery catheter 100,100A to adjust an angle (as shown in phantom) of the distal tip of the delivery catheter relative to the longitudinal length of the shaft 104, 104A. As schematically shown, the length of the control wire 340 extends from the distal tip to the proximal end of the catheter shaft 104 or hub so that the user can intravenously adjust the angle of the distal tip to steer the delivery catheter 100, 100A to the treatment or imaging site via movement of the control wire(s) 340 as illustrated by arrows 342. In illustrated embodiments, one or more control wires 340 extend along one or more control wire lumens (not shown) formed along the catheter shaft 104 and illustrative embodiments include one or more control wires 340 and one or more control wire lumens (not shown).

## Claims

1. An intracardiac delivery catheter (100A) for intracardiac placement of an intracardiac echocardiography (ICE) catheter (102) comprising:
a catheter shaft (104A) having an elongate length extending along a longitudinal axis of the catheter shaft between a proximal end (106) and a distal end (108) of the catheter shaft and a radial dimension, the length and radial dimension sized for intravascular insertion and the catheter shaft having a flexible distal portion (222) bendable for intracardiac insertion of the catheter shaft through a right atrium, right ventricle into a pulmonary artery and a relatively stiff proximal length (220) for torquability of the catheter shaft;
a delivery lumen (130) formed along the elongate length of the catheter shaft (104A) having an elongate length extending between an inlet and an outlet at the distal end of the catheter shaft and sized for insertion of the ICE catheter (102) therethrough;
an asymmetric flow assist balloon (110A) coupled to the catheter shaft proximate to the distal end of the catheter shaft (104A), the balloon (110A) having an asymmetric shape along a radial axis generally transverse to the longitudinal axis of the catheter shaft when inflated to form a moment arm proximate to the distal end of the catheter shaft;_and
an inflation lumen (134) formed along the elongate length of the catheter shaft (104A) having an inlet port connectable to an inflation device (112) and an outlet port opened to an interior of the asymmetric flow assist balloon (110A) to inflate the balloon to provide the asymmetric shape orientatable through the torqueable catheter shaft to form the moment arm to bend the distal end (108) of the catheter shaft (104A) to steer the catheter through the right atrium, right ventricle into the pulmonary artery utilizing fluid flow.

2. The catheter of claim 1, wherein the outlet for the delivery lumen (130) is distally spaced from the asymmetric flow assist balloon (110A).

3. The catheter of claim 1, wherein the outlet of the delivery lumen (130) is formed through an opened distal end of the catheter shaft.

4. The catheter of claim 3, wherein the delivery lumen (130) has an inner diameter dimension of at least 8F.

5. The device of claim 3, wherein the delivery lumen (130) has a diameter dimension of at least 9F.

6. The catheter device of claim 1, wherein the catheter shaft (104A) further comprises a guide wire lumen (132) for insertion of a guide wire (120).

7. The catheter device of claim 6, wherein the guidewire lumen has a shortened length formed between a proximal opening (202A) spaced from the proximal end of the catheter shaft (104A) and a distal opening (204A) spaced from the proximal opening (202A).

8. The catheter of claim 1 in combination with the intracardiac echocardiography catheter including an echocardiography transducer array (166).

9. The catheter of claim 1 wherein the delivery lumen (130) has an inner balloon (192) inflatable through an inner inflation lumen (190) to retain the ICE catheter (102) in place in the delivery lumen (130) and provide an ultrasonic transmission fluid or medium.

10. The catheter of claim 1 wherein the catheter shaft (104A) includes a transition zone between the relatively stiff proximal length (220) and the flexible distal portion (222).

11. The catheter of claim 1 wherein the flexible distal portion (222) is formed of a helical coil embedded within a "soft" polymer body or layers.

## Patentansprüche

1. Intrakardialer Einführkatheter (100A) zur intrakardialen Platzierung eines Katheters (102) für die intrakardiale Echokardiographie (ICE), umfassend:
einen Katheterschaft (104A) mit einer Längsausdehnung, die sich entlang einer Längsachse des Katheterschafts zwischen einem proximalen Ende (106) und einem distalen Ende (108) des Katheterschafts erstreckt, sowie einer radialen Abmessung, wobei die Längsausdehnung und die radiale Abmessung für die intravaskuläre Einführung ausgelegt sind und der Katheterschaft einen flexiblen distalen Abschnitt (222) aufweist, der zum intrakardialen Einführen des Katheterschafts durch den rechten Vorhof, den rechten Ventrikel in eine Lungenarterie sowie einen relativ steifen proximalen Abschnitt (220) zur Drehbarkeit des Katheterschafts;
ein entlang der Längsausdehnung des Katheterschafts (104A) ausgebildetes Einführlumen (130), dessen Längsachse sich zwischen einem Einlass und einem Auslass am distalen Ende des Katheterschafts erstreckt und das so dimensioniert ist, dass der ICE-Katheter (102) hineingeführt werden kann;
einen asymmetrischen Strömungsunterstützungsballon (110A), der nahe dem distalen Ende des Katheterschafts (104A) mit diesem verbunden ist, wobei der Ballon (110A) im aufgeblasenen Zustand eine asymmetrische Form entlang einer Radialachse aufweist, die im Wesentlichen quer zur Längsachse des Katheterschafts verläuft, um nahe dem distalen Ende des Katheterschafts einen Hebelarm zu bilden; und
ein Aufblaslumen (134), das entlang der Längsausdehnung des Katheterschafts (104A) ausgebildet ist und eine Einlassöffnung aufweist, die mit einer Aufblasvorrichtung (112), und eine Auslassöffnung, die zum Inneren des asymmetrischen Strömungsunterstützungsballons (110A) hin offen ist, um den Ballon aufzublasen und so die asymmetrische Form bereitzustellen, die über den drehbaren Katheterschaft ausrichtbar ist, um den Hebelarm zu bilden, der das distale Ende (108) des Katheterschafts (104A) biegt, um den Katheter unter Ausnutzung der Flüssigkeitsströmung durch den rechten Vorhof, den rechten Ventrikel in die Lungenarterie zu lenken.

2. Katheter nach Anspruch 1, wobei der Auslass des Einführlumens (130) distal vom asymmetrischen Strömungsunterstützungsballon (110A) beabstandet ist.

3. Katheter nach Anspruch 1, wobei der Auslass des Einführlumens (130) durch ein offenes distales Ende des Katheterschafts gebildet ist.

4. Katheter nach Anspruch 3, wobei das Einführlumen (130) einen Innendurchmesser von mindestens 8F aufweist.

5. Vorrichtung nach Anspruch 3, wobei das Einführlumen (130) einen Durchmesser von mindestens 9E aufweist.

6. Kathetervorrichtung nach Anspruch 1, wobei der Katheterschaft (104A) ferner ein Führungsdrahtlumen (132) zum Einführen eines Führungsdrahts (120) umfasst. .

7. Kathetervorrichtung nach Anspruch 6, wobei das Führungsdrahtlumen eine verkürzte Länge aufweist, die zwischen einer proximalen Öffnung (202A), die vom proximalen Ende des Katheterschafts (104A) beabstandet ist, und einer distalen Öffnung (204A), die von der proximalen Öffnung (202A) beabstandet ist, gebildet wird.

8. Katheter nach Anspruch 1 in Kombination mit dem intrakardialen Echokardiographiekatheter, der eine Echokardiographie-Wandleranordnung (166) umfasst.

9. Katheter nach Anspruch 1, wobei das Einführlumen (130) einen inneren Ballon (192) aufweist, der über ein inneres Aufblaslumen (190) aufblasbar ist, um den ICE-Katheter (102) im Einführlumen (130) in Position zu halten und eine Ultraschallübertragungsflüssigkeit oder ein Ultraschallübertragungsmedium bereitzustellen.

10. Katheter nach Anspruch 1, wobei der Katheterschaft (104A) eine Übergangszone zwischen dem relativ steifen proximalen Abschnitt (220) und dem flexiblen distalen Abschnitt (222) umfasst.

11. Katheter nach Anspruch 1, wobei der flexible distale Abschnitt (222) aus einer spiralförmigen Spule gebildet ist, die in einen "weichen" Polymerkörper oder in Polymerlagen eingebettet ist.

## Revendications

1. Cathéter de placement intracardiaque (100A) destiné au placement intracardiaque d'un cathéter intracardiaque d'échocardiographie (ICE) (102) comprenant :
une tige de cathéter (104A) présentant une longueur allongée s'étendant le long d'un axe longitudinal de la tige du cathéter entre une extrémité proximale (106) et une extrémité distale (108) de la tige du cathéter et une dimension radiale, la longueur et la dimension radiale étant dimensionnées pour une insertion vasculaire et la tige de cathéter présentant une partie distale flexible (222) pliable pour l'insertion intracardiaque de la tige de cathéter à travers une oreillette droite puis dans le ventricule droit jusqu'à l'artère pulmonaire, ainsi qu'une partie proximale (220) relativement rigide permettant d'appliquer un couple à la tige du cathéter ;
une lumière de placement (130) formée le long de la longueur allongée de la tige du cathéter (104A) présentant une longueur allongée s'étendant entre une entrée et une sortie au niveau de l'extrémité distale de la tige du cathéter et dimensionnée pour une insertion du cathéter ICE (102) à travers celle-ci ;
un ballon d'assistance d'écoulement asymétrique (110A) couplé à la tige du cathéter à proximité de l'extrémité distale de la tige du cathéter (104A), le ballon (110A) présentant une forme asymétrique le long d'un axe radial généralement transversal à l'axe longitudinal de la tige du cathéter lorsqu'il est gonflé pour former un bras de levier à proximité de l'extrémité distale de la tige du cathéter ; et
une lumière de gonflage (134) formée le long de la longueur allongée de la tige du cathéter (104A) présentant un port d'entrée connectable à un dispositif de gonflement (112) et un port de sortie s'ouvrant sur un intérieur du ballon d'assistance d'écoulement asymétrique (110A) afin de gonfler le ballon pour fournir la forme asymétrique orientable à travers la tige du cathéter capable de générer un couple, de manière à former le bras de levier pour plier l'extrémité distale (108) de la tige du cathéter (104A) pour diriger le cathéter à travers l'oreillette droite, puis le ventricule droit, dans l'artère pulmonaire en utilisant un écoulement de fluide.

2. Cathéter selon la revendication 1, selon lequel la sortie de la lumière de placement (130) est espacée distalement du ballon d'assistance d'écoulement asymétrique (110A).

3. Cathéter selon la revendication 1, selon lequel la sortie de la lumière de placement (130) est formée à travers une extrémité distale ouverte de la tige du cathéter.

4. Cathéter selon la revendication 3, selon lequel la lumière de placement (130) présente une dimension de diamètre intérieur d'au moins 8F.

5. Dispositif selon la revendication 3, selon lequel la lumière de placement (130) présente une dimension de diamètre d'au moins 9E.

6. Dispositif de cathéter selon la revendication 1, selon lequel la tige du cathéter (104A) comprend en outre une lumière de fil-guide (132) pour l'insertion d'un fil-guide (120).

7. Dispositif de cathéter selon la revendication 6, selon lequel la lumière de fil-guide présente une longueur raccourcie formée entre une ouverture proximale (202A) espacée de l'extrémité proximale de la tige du cathéter (104A) et une ouverture distale (204A) espacée de l'extrémité proximale (202A).

8. Cathéter selon la revendication 1 en combinaison avec le cathéter intracardiaque d'échocardiographie, comportant un réseau de transducteurs d'échocardiographie (166).

9. Cathéter selon la revendication 1, selon lequel la lumière de placement (130) présente un ballon intérieur (192) gonflable à travers une lumière de gonflage intérieure (190) afin de retenir le cathéter ICE (102) en place dans la lumière de placement (130) et afin de fournir un fluide ou médium de transmission d'ultrasons.

10. Cathéter selon la revendication 1, selon lequel la tige du cathéter (104A) comporte une zone de transition entre la longueur proximale relativement rigide (220) et la partie distale flexible (222).

11. Cathéter selon la revendication 1, selon lequel la partie distale flexible (222) est formée d'une boucle hélicoïdale encapsulée par un corps ou des couches de polymère "doux".
